(19) Europäisches Patentamt
European Patent Office
Office européen des brevets

(11) **EP 3 608 668 B1**

(12) **EUROPEAN PATENT SPECIFICATION**

(45) Date of publication and mention
of the grant of the patent:
**06.01.2021 Bulletin 2021/01**

(51) Int Cl.:
***G01N 33/52*** *(2006.01)*

(21) Application number: **18187931.3**

(22) Date of filing: **08.08.2018**

(54) **DEVICES AND METHOD FOR MEASURING AN ANALYTE CONCENTRATION IN BLOOD**

VORRICHTUNGEN UND VERFAHREN ZUM MESSEN EINER ANALYTKONZENTRATION IN EINER PROBE BLUT

DISPOSITIFS ET PROCÉDÉ POUR MESURER UNE CONCENTRATION D'ANALYTE DANS UN ÉCHANTILLON DE SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.02.2020 Bulletin 2020/07**

(73) Proprietors:
• **Roche Diabetes Care GmbH**
**68305 Mannheim (DE)**
Designated Contracting States:
**DE**
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**

(72) Inventors:
• **TUERCK, Volker**
**D-10965 Berlin (DE)**
• **BERG, Max**
**68305 Mannheim (DE)**
• **SIEFFERT, Daniel**
**68305 Mannheim (DE)**

(74) Representative: **Altmann Stößel Dick**
**Patentanwälte PartG mbB**
**Dudenstrasse 46**
**68167 Mannheim (DE)**

(56) References cited:
**WO-A1-2013/158505      US-A1- 2005 109 951**
**US-A1- 2006 051 738      US-A1- 2017 343 480**
**US-A1- 2018 172 591**

## Description

Technical Field

The present invention relates to an optical test strip as well as to a kit for measuring an analyte concentration in a sample of blood. The invention further relates to a method for measuring an analyte concentration in a sample of blood optical test strips, kits and methods according to the present invention may be used in medical diagnostics, in order to quantitatively or qualitatively detect and/or measure a concentration of one or more analytes in one or more bloods. Other fields of application of the present invention are also feasible.

Background art

In the field of medical diagnostics, in many cases, concentrations of one or more analytes in samples of body fluids, such as blood, interstitial fluid, urine, saliva or other types of bodily fluids have to be detected and/or measured. Examples of analytes to be detected are glucose, triglycerides, lactate, cholesterol or other types of analytes typically present in these body fluids. According to the concentration and/or the presence of the analyte, an appropriate treatment may be chosen, if necessary.

Generally, devices and methods known to the skilled person make use of test elements comprising one or more test chemistries, which, in presence of the analyte to be detected, are capable of performing one or more detectable detection reactions, such as optically detectable detection reactions.

Typically, one or more optically detectable changes in the test chemistry are monitored, in order to derive the concentration of the at least one analyte to be detected from these changes. For detecting the at least one change of optical properties of the test chemistry, various types of detectors are known in the art. In recent developments, consumer-electronics such as mobile phones, laptops, smartphones and other portable devices have become popular to be used as detectors for detecting the changes in the test chemistry. Besides using consumer-electronics for detecting the changes of optical properties of the test chemistry in common test strips, acquiring information from specially designed test modules by using consumer-electronics, e.g. a camera of a portable device, are also known from the art. Thus, US 2017/0343480 A1 discloses a method for measuring blood glucose levels by a portable terminal using a strip module. The strip module includes a dye pad having a color that changes in response to a sample applied to the dye pad. The strip module also includes a transparent strip having a first side and a second side. The first side is opposite the second side. The dye pad is mounted on the first side of the transparent strip, and the transparent strip reflects light provided from a light source of a portable terminal located adjacent to the second side and transmits the light to the dye pad.

Further, in US 2006/051738 A1 diagnostic dry reagent tests capable of reacting with a single drop of whole blood and reporting both glucose and lightscattering analytes, such as chylomicrons are described. Such dry reagent tests may employ electrochemical detection methodologies, optical detection methodologies, or both methodologies. These tests alert diabetics to excessive levels of postprandial lipemia caused by meals with excessive amounts of fat, and thus can help reduce the risk of cardiovascular complications in diabetic patients.

However, despite the advantages involved in using consumer-electronics for the purpose of measuring an analyte concentration in samples of bodily fluid, several technical challenges remain. Specifically, ambient light may contribute significantly to the light detected by a camera of the mobile device, such as a smartphone camera. Thus, the impact of ambient light on the determined analyte concentration generally needs to be considered which, so far, requires complex combinations of lighting arrangements, additional coupling means and specially designed test strips, such as for example known from US 2017/0343480 A1. In particular, the common approach of considering impact of ambient light by using additional hardware, generally leads to significant inconvenience for the user and an increase of the economic burden.

Problem to be solved

It is therefore desirable to provide devices and methods which address the above mentioned technical challenges of analytical measurements. Specifically, an optical test strip, a kit and a method shall be provided which lessen the impact of ambient light when determining or measuring an analyte concentration in a sample of blood, without requiring additional hardware.

Summary

This problem is addressed by an optical test strip, a kit and method for measuring an analyte concentration in a sample of blood with the features of the independent claims. Advantageous embodiments which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, an optical test strip for measuring an analyte concentration in a sample of blood is disclosed. The optical test strip comprises a test strip carrier having at least one transparent area and a test field. The test field comprises at least one carrier foil, at least one test chemical applied to the carrier foil and at least one porous material.

As used herein, the term "optical test strip" is a broad term and is to be given its ordinary and customary meaning to a person of ordinary skill in the art and is not to be limited to a special or customized meaning. The term specifically may, without limitation, refer to an arbitrary element configured for measuring an analyte concentration in a sample of blood. The optical test strip may particularly be configured for performing a color-change detection reaction and thereby providing optically detectable information on the analyte concentration. As an example, the optical test strip may particularly be strip shaped, thus, the test strip may have a long and narrow shape.

The term "analyte" as used herein is a broad term and is to be given its ordinary and customary meaning to a person of ordinary skill in the art and is not to be limited to a special or customized meaning. The term specifically may refer, without limitation, to one or more specific chemical compounds and/or other parameters to be detected and/or measured. As an example, the at least one analyte may be a chemical compound which takes part in metabolism, such as one or more of glucose, cholesterol or triglycerides. Additionally or alternatively, other types of analytes or parameters may be determined, e.g. a pH value.

The term "measuring an analyte concentration in a sample" as used herein is a broad term and is to be given its ordinary and customary meaning to a person of ordinary skill in the art and is not to be limited to a special or customized meaning. The term specifically may refer, without limitation, to a quantitatively and/or qualitatively determination of at least one analyte in an arbitrary sample. For example, the sample may comprise a body fluid, such as blood, interstitial fluid, urine, saliva or other types of body fluids. The result of the measurement, as an example, may be a concentration of the analyte and/or the presence or absence of the analyte to be measured. Specifically, as an example, the measurement may be a blood glucose measurement, thus the result of the measurement may for example be a blood glucose concentration.

The term "test strip carrier" as used herein is a broad term and is to be given its ordinary and customary meaning to a person of ordinary skill in the art and is not to be limited to a special or customized meaning. The term specifically may refer, without limitation, to an arbitrary substrate configured to provide stabilizing means to the optical test strip, specifically to the test field. The test strip carrier specifically may have a strip-shape, e.g. a shape of a rectangular strip. The test strip carrier, as an example, may be flexible and/or deformable. The test strip carrier, as an example, may have a width, e.g. a lateral extension perpendicular to a longitudinal axis of the test strip, of 1 mm to 20 mm, e.g. 2 mm to 5 mm. The test strip carrier further may have a length, e.g. a longitudinal extension of 10 mm to 70 mm, e.g. 15 mm to 50 mm. The length may exceed the width by e.g. a factor of at least 1.5. The test strip carrier further may have a thickness of 100 micrometers to 2 mm, e.g. 500 micrometers to 1 mm. The test strip carrier may fully or partially be made of at least one material such as one or more of a plastic material, a ceramic material or a paper. Specifically, the test strip carrier may fully or partially be made of at least one plastic foil. The test strip carrier may be made of a single layer or of a plurality of layers. The test strip carrier specifically may be opaque, such as by comprising at least one material which is fully or partially intransparent for visible light.

The test strip carrier has at least one transparent area, such as for example an area fully or partially made of a translucent material or an area having at least one opening, breakthrough or hole in the test strip carrier. The transparent area, as an example, may have a round, oval or polygonal shape. The transparent area, as an example, may fully or partially be surrounded by intransparent or opaque material of the test strip carrier. The transparent area, as an example, may form at least one window, specifically a window opening, in the test strip carrier. The window or window opening specifically, as will be outlined in further detail below, may fully or partially be covered by the test field, which, as an example, may be applied to the test strip carrier in the region of the at least one transparent area, thereby, e.g., covering at least the window. However, the transparent area may for example expand over the whole test strip, such as cover the test strip completely. Thus, in particular, the test strip carrier itself may for example be fully made of a transparent material and may therefore, for example, itself be the transparent area.

The term "test field" as used herein is a broad term and is to be given its ordinary and customary meaning to a person of ordinary skill in the art and is not to be limited to a special or customized meaning. The term specifically may refer, without limitation, to an arbitrary element having at least one amount of a test chemical for detecting at least one analyte. The test field, as an example, may comprise at least one layer comprising the test chemical. As an example, the test field may comprise an arbitrary layered element, having a layered structure, with the test chemical being comprised by at least one layer of the layered structure. Particularly, the term may refer to a coherent amount of the test chemical, such as to a field, e.g. a field of round, polygonal or rectangular shape, having one or more layers of material, with at least one layer of the test field, such as the carrier foil, having the test chemical applied thereto. Other layers may be present providing spreading properties for spreading the sample or providing separation properties such as for separating of particulate components of the sample, such as cellular components, for example by comprising the at least one porous material.

In particular, the test field comprises the at least one porous material, for example material being fully or partially porous, for at least partially filtering out solid components contained in the sample. The porous material in particular may be configured for separating particulate or solid components of the sample. Thus, the porous material may specifically be or may comprise a filter material, such as for example titanium dioxide ($TiO_2$). In particular, the porous material may for example filter out cellular components comprised in the sample of blood.

Further, the test field comprises the at least one carrier foil. The at least one carrier foil of the test field is applied to the test strip carrier and covers the transparent area of the test strip carrier. Thus, the carrier foil may for example cover or overlap the transparent area, for example an opening or hole, of the test strip carrier. Specifically, the carrier foil may be or may comprise a material having an inherent rigidity so as to be suitable for covering the transparent area, such as the opening or hole, of the test strip carrier.

The term "carrier foil" as used herein is a broad term and is to be given its ordinary and customary meaning to a person of ordinary skill in the art and is not to be limited to a special or customized meaning. The term specifically may refer, without limitation, to an arbitrary film like material. Specifically, the carrier foil may have a foil shape wherein the carrier foil in a first extension direction may be at least ten times smaller than an extension of the carrier foil in another direction, extending orthogonally to the first direction. The carrier foil specifically may be made of at least one flexible or deformable material, such as at least one flexible or deformable plastic foil. The plastic foil, as an example, may have a thickness of 10 micrometers to 500 micrometers. The carrier foil, specifically, may comprise at least one transparent matrix material, such as at least one transparent plastic material being translucent in the visible spectral range. Examples will be given in further detail below.

In particular, the carrier foil may comprise a complex structure, for example a layered structure having one or more layers of material. Thus, the carrier foil may specifically comprise the at least one layer of transparent matrix material. Other layers may be present, for example adhesive layers, such as glue layers, adhesive tape layers, or other layers for bonding.

The carrier foil further has at least one wavelength filter component which is adapted to essentially block light having wavelengths $\lambda_{blc}$ of $10\,nm \leq \lambda_{blc} \leq WL_{low}$, with $550\,nm \leq WL_{low} \leq 650\,nm$. In particular, $WL_{low}$ refers to a wavelength characterizing the at least one wavelength filter component. The term "light" as used herein is a broad term and is to be given its ordinary and customary meaning to a person of ordinary skill in the art and is not to be limited to a special or customized meaning. The term specifically may refer, without limitation, to an electromagnetic radiation having wavelengths within an electromagnetic spectrum. Specifically, the term light as referred to hereinafter, may specifically be or may comprise electromagnetic radiation having wavelengths $\lambda_e$ at least in the range of $10\,nm \leq \lambda_e \leq 1200\,nm$, particularly $100\,nm \leq \lambda_e \leq 1200\,nm$, more particularly $250\,nm \leq \lambda_e \leq 1200\,nm$, even more particularly $400\,nm \leq \lambda_e \leq 1200\,nm$.

In particular, the wavelength filter component may for example be introduced into or mixed within a matrix material of the carrier foil, e.g. a transparent matrix material, of the carrier foil, specifically within at least one layer of the carrier foil. Additionally or alternatively, the wavelength filter component may be implemented into the matrix material by being one or more of dispersed in the matrix material or chemically bound to the matrix material, e.g. by covalent bond, chemical complexing or ion bonding. Additionally or alternatively, the wavelength filter component may also form at least one filter layer, e.g. at least one layer disposed on one or both sides of at least one layer of the matrix material.

The term "essentially block" as used herein is a broad term and is to be given its ordinary and customary meaning to a person of ordinary skill in the art and is not to be limited to a special or customized meaning. The term specifically may refer, without limitation, to a process of a majority of electromagnetic radiation being stopped or blocked from passing through matter. In particular, the wavelength filter component having the characterizing wavelength $WL_{low}$ and configured for essentially blocking light having wavelengths $\lambda_{blc}$, may specifically be configured for one or both of absorbing or reflecting $\geq 80\ \%$ of the intensity of electromagnetic radiation having wavelengths $\lambda_{blc} \leq WL_{low}$, from transmitting or passing through the carrier foil. Thus, the wavelength filter component having the characterizing wavelength $WL_{low}$ and configured for essentially blocking light having wavelengths $\lambda_{blc}$, may specifically be configured for transmitting less than 20 %, in particular less than 10 %, more particular less than 5 %, of light having wavelengths $\lambda_{blc} \leq WL_{low}$ through the carrier foil. The transmission may specifically be defined as a quotient of an intensity of light, e.g. electromagnetic radiation, transmitted by the filter, divided by the starting intensity of the light incident on the filter, multiplied by 100 %.

The blocking effect of the at least one wavelength filter component may be based on various physical principles. Thus, as an example, the wavelength filter component may comprise at least one filter material being suited for absorbing the light, specifically in a wavelength-selective fashion, such as at least one dye, e.g. at least one organic or inorganic dye. The filter material, e.g. the at least one dye, as an example, may be introduced in at least one matrix material, e.g. as outlined above. Additionally or alternatively, the filter material may also be comprised by at least one filter layer, e.g. at least one layer of the filter material being directly or indirectly applied onto one or both sides of the carrier foil. Further, in addition or as an alternative to an absorption, the blocking effect may be also achieved by a reflection, e.g. in a wavelength-selective fashion. Thus, as an example and as will be outlined in further detail below, the wavelength filter component may comprise at least one multi-layer setup comprising a plurality of layers having differing optical refractive indices. Thus, as an example, the wavelength filter component may comprise at least one interference filter, e.g. at least one interference filter having a plurality of layers of at least one organic or inorganic material, the layers having a varying refractive index, e.g. a periodically varying refractive index. The layer setup, as an example, may directly or indirectly be applied to the carrier foil on one or both sides. Additionally or alternatively, the carrier foil itself may be part of the wavelength-selective element. Combinations of the named possibilities are feasible.

The test field further comprises at least one test chemical directly or indirectly applied to the carrier foil. The test chemical is configured for performing an optically detectable detection reaction with the analyte. The term "test chemical" as used herein is a broad term and is to be given its ordinary and customary meaning to a person of ordinary skill in the art and is not to be limited to a special or customized meaning. The term specifically may refer, without limitation, to a chemical compound or a plurality of chemical compounds such as a mixture of chemical compounds suited for performing a detection reaction in the presence of the analyte, wherein the detection reaction is detectable by specific means, such as optically. The detection reaction specifically may be analyte-specific. The test chemical, in the present case, specifically may be an optical test chemical, such as a color-change test chemical which changes in color in the presence of the analyte. The color change specifically may depend on the amount of analyte present in the sample. The test chemical, as an example, may comprise at least one enzyme, such as glucose oxidase and/or glucose dehydrogenase. Additionally, other components may be present, such as one or more dyes, mediators and the like. Test chemicals are generally known to the skilled person and reference may be made to J. 20 Hones et al.: Diabetes Technology and Therapeutics, Vol. 10, Supplement 1, 2008, pp.10-26. Other test chemicals, however, are feasible, too.

The test chemical is further configured for at least partially, for example fully or partially, absorbing light having at least one absorption wavelength $\lambda_{abs}$ in the range 650 nm $< \lambda_{abs} \leq 1100$ nm. In particular, light having the at least one absorption wavelength $\lambda_{abs}$ may in particular be fully or partially absorbed by the test chemical. The term "absorb" as used herein is a broad term and is to be given its ordinary and customary meaning to a person of ordinary skill in the art and is not to be limited to a special or customized meaning. The term specifically may refer, without limitation, to a process of energy being taken up by matter, such as the electrons of an atom. Thus, in particular, electromagnetic energy of light having the at least one absorption wavelength $\lambda_{abs}$ may be at least partially taken up by the test chemical and thereby for example be transformed into internal energy of the test chemical. Thus, as an example, the test chemical may specifically have an extinction or attenuation coefficient $\alpha > 0$.

As an example, for the blocking wavelength $\lambda_{blc}$ of the wavelength filter component adapted to essentially block light having wavelengths $\lambda_{blc}$, may specifically apply 10 nm $\leq \lambda_{blc} \leq WL_{low}$, e.g. 100 nm $\leq \lambda_{blc} \leq WL_{low}$, 250 nm $\leq \lambda_{blc} \leq WL_{low}$, or 400 nm $\leq \lambda_{blc} \leq WL_{low}$. Thus, in particular, the wavelength filter component may be adapted to essentially block all light from an ultraviolet range to $WL_{low}$. Specifically, the wavelength filter component may, for example, be configured to essentially block ultraviolet (UV) light, such as electromagnetic radiation in the UV range, as well as visible light, specifically electromagnetic radiation visible for human eyes, below or equal to $WL_{low}$.

The wavelength filter component, in particular, may be located within the carrier foil. Specifically, the wavelength filter component may, for example, be dispersed within the carrier foil, such as mixed within the material of the carrier foil.

As an example, the wavelength filter component may be selected from the group consisting of a longpass filter component and a bandpass filter component. Specifically, the wavelength filter component may specifically be or may comprise a longpass filter, such as for example the wavelength filter component may be configured for essentially block light having wavelengths $\lambda_{blc} \leq WL_{low}$. Alternatively, the wavelength filter component may be or may comprise a bandpass filter. The bandpass filter may specifically be or may comprise a combination of a longpass filter and a shortpass filter and may thus only transmit light within a predefined wavelength range, for example only within a wavelength band. Thus, in particular, the wavelength filter component may additionally be configured to block light having wavelengths $\lambda_{blc} \geq$ WL$_{high}$. Specifically, WL$_{high}$ may refer to an additional wavelength further characterizing the at least one wavelength filter component. As an example, the wavelength filter component may be configured to essentially block light for example having a wavelength of WL$_{high}$ and higher, as well as light having wavelengths WL$_{low}$ and lower.

In particular, the wavelength filter component may specifically be or may comprise at least one longpass filter. The longpass filter may particularly have a transmission edge rising with the wavelength of the light. Thus, the longpass filter may specifically show a higher transmission of light, the higher the wavelength. In particular, the transmission of light through the longpass filter may rise with rising wavelength. Further, the longpass filter may have a characterizing wavelength $\lambda_{LP}$. Thus, WL$_{low}$ may equal $\lambda_{LP}$. In particular, a transmission $T_{LP}$ of the longpass filter at $\lambda_{LP}$ may be 50 % of a maximum transmission $T_{LPmax}$ of the longpass filter. Thus, the characterizing wavelength $\lambda_{LP}$ may be defined such that a transmission $T_{LP}$ of the longpass filter at $\lambda_{LP}$ may be 50 % of the maximum transmission $T_{LPmax}$ of the longpass filter. In particular, as an example, if the longpass filter, for example in its transmission range, has a maximum transmission of 85 %, the characteristic wavelength $\lambda_{LP}$ for this case is defined as that wavelength at which the longpass filter attains a transmission of 0.5 x 85 % = 42.5 %, for example when viewing the transmission spectrum with rising wavelengths. In particular, the maximum transmission of the longpass filter may for example be at least 75 %, specifically at least 80 %, more specifically at least 85 % or even at least 90 % or at least 95 %.

Further, the longpass filter may have a steepness $S_{LP}$ of the rising transmission edge. In particular, it may be preferred when the longpass filter has a steep transmission edge in order to block or absorb a maximum part of light having wavelengths below $\lambda_{LP}$ and a maximum part of light having wavelengths over or above $\lambda_{LP}$. The steepness of the longpass filter may generally be reported in the unit electron volts (eV) and may be defined as

$$S_{LP} = h \cdot c \cdot [(1/\lambda_{blc}) - (1/\lambda_{trans})]. \qquad (1)$$

In Equation (1), $\lambda_{blc}$ may specifically be that wavelength at and below which the longpass filter essentially blocks light. Thus, at wavelength $\lambda_{blc}$ the Transmission $T_{LP}$ of the longpass filter may specifically be smaller than 20 %, in particular smaller than 10 %, more particular smaller than 5 %. Further, $\lambda_{trans}$ may be defined as being that wavelength at and above which the longpass filter attains a value of 95 % of the maximum transmission $T_{LPmax}$ of the longpass filter. Thus, at wavelengths smaller than $\lambda_{trans}$ the transmission $T_{LP}$ of the longpass filter may be < 95 % of the maximum transmission $T_{LPmax}$ of the longpass filter and at wavelengths equal or greater than $\lambda_{trans}$ the transmission $T_{LP}$ may be $\geq$ 95 % of $T_{LPmax}$, for example 95 % to 100 % of $T_{LPmax}$. If, for example, the longpass filter, more particularly in a transmission region, has a maximum transmission of 85 %, $\lambda_{trans}$ may be defined as that wavelength at which, for example with rising wavelength, the transmission attains a value of 0.95 x 85 % = 80.75 %. In addition, the above mentioned formula for the steepness of the longpass filter, the parameter h denotes Planck's constant (h $\approx$ 6.626 . 10$^{-34}$ Js) and c the speed of light in a vacuum (c $\approx$ 3.0 . 10$^8$ m/s). With steepness defined in such a way, specifically, the steepness $S_{LP}$ may for example be 0 eV < $S_{LP} \leq$ 1.2 eV, specifically 0.1 eV $\leq S_{LP} \leq$ 1.1 eV, more specifically 0.2 eV $\leq S_{LP} \leq$ 0.9 eV.

In particular, the characterizing wavelength WL$_{low}$ characterizing the at least one wavelength filter component, may for example be in the range of 600 nm $\leq$ WL$_{low} \leq$ 650 nm, more specifically in the range of 625 nm $\leq$ WL$_{low} \leq$ 650 nm.

The test field of the optical test strip may particularly have a shape selected from the group consisting of: a rectangular shape; a square shape; a round shape; a circular shape. Further, the test field may comprise at least one spreading layer. In particular, the spreading layer may be configured to equally spread or distribute the sample of blood over a surface of the test field on which the sample may be applied.

The wavelength filter may for example comprise an interference filter, specifically a high-pass interference filter. The term "interference filter" as used herein is a broad term and is to be given its ordinary and customary meaning to a person of ordinary skill in the art and is not to be limited to a special or customized meaning. The term specifically may refer, without limitation, to an optical filter that reflects one or more spectral bands or lines and transmits others, while maintaining a nearly zero coefficient of absorption for all wavelengths of interest. As an example, the interference filter may comprise multiple layers of dielectric material having different refractive indices. In particular, the interference filter comprises wavelength selective properties. Thus, as an example, the high-pass interference filter having a characteristic wavelength $\lambda_{HPF}$ also referred to as cutoff frequency, may selectively block or attenuate all light having wavelengths below $\lambda_{HPF}$, wherein the high-pass interference filter may transmit all light having wavelengths higher than X$_{HPK}$.

The interference filter may specifically be located on at least one surface of the carrier foil. As an example, the interference filter may be directly or indirectly applied to an upper surface of the carrier foil, for example as a separate layer. Additionally or alternatively, the interference filter may be directly or indirectly applied to a lower surface of the carrier foil. Thus, the interference filter may for example be located on both the upper and the lower surface of the carrier foil.

Further, the optical test strip, specifically the carrier foil, may comprise at least one further filter component. In particular, the at least one further filter component may comprise a shortpass filter. Specifically, the shortpass filter may have a transmission edge falling with the wavelength of the light. Thus, the shortpass filter may specifically show an increasing transmission of light for decreasing wavelengths. In particular, the transmission of light through the shortpass filter may fall with rising wavelength. Further, the shortpass filter may have a characteristic wavelength $\lambda_{SP}$, wherein $\lambda_{SP}$ may equal $WL_{high}$. In particular, a transmission $T_{SP}$ of the shortpass filter at $\lambda_{SP}$ may be 50 % of a maximum transmission $T_{SPmax}$ of the shortpass filter. For example, the characteristic wavelength $\lambda_{SP}$ of the shortpass filter may be in the range of 630 nm $\leq \lambda_{SP} \leq$ 800 nm, specifically in the range of 640 nm $\leq \lambda_{SP} \leq$ 680 nm.

As an example, the further filter component, specifically the shortpass filter, may be or may comprise a short-pass interference filter. Specifically, the short-pass interference filter may for example be an interference filter as defined above. In particular, the short-pass interference filter may comprise multiple layers of dielectric material having different refractive indices. In particular, the short-pass interference filter may also comprise wavelength selective properties. Thus, as an example, the short-pass interference filter may have a characteristic wavelength $\lambda_{SPF}$ and may selectively block or attenuate all light having wavelengths higher than $\lambda_{SPF}$, wherein the short-pass interference filter may transmit all light having wavelengths lower than $\lambda_{SPF}$.

The optical test strip, specifically the carrier foil, may for example comprise a combination of filter components. As an example, the optical test strip may comprise a combination of a longpass filter and a shortpass filter, e.g. a high-pass interference filter and a short-pass interference filter. However, other combinations of filters are feasible.

In particular, the further filter component may be configured for essentially blocking transmission of light having wavelengths $\lambda \geq WL_{high}$, with $WL_{high} > WL_{low}$, specifically $WL_{high} \geq WL_{low} + 20$ nm, more specifically $WL_{high} \geq WL_{low} + 30$ nm, e.g. $WL_{low} + 20$ nm $\leq WL_{high} \leq WL_{low} + 60$ nm, e.g. $WL_{low} + 30$ nm $\leq WL_{high} \leq WL_{low} + 50$ nm.

In particular, the carrier foil may for example comprise at least one material selected from the group consisting of: a thermoplastic material; a Polyethylene terephthalate (PET); a polyethylene glycol (PEG); a polycarbonate (PC), specifically Pokalon®; a polypropylene (PP), a polystyrene (PS). Further, as an example, the test strip carrier may comprise at least one material selected from the group consisting of: a plastic material; a thermoplastic material; a polycarbonate, specifically Makrolon® or Lexan®.

The optical test strip, for example, may further comprise at least one reference color field. The term "reference color field" as used herein is a broad term and is to be given its ordinary and customary meaning to a person of ordinary skill in the art and is not to be limited to a special or customized meaning. The term specifically may refer, without limitation, to an arbitrary two-dimensional area which has a predetermined color of known properties. In particular, the reference color field may for example comprise at least one white field, such as a field having a white color. Further, the reference color field may have a shape which is selected from the group consisting of: a rectangular shape; a square shape; a round shape; a circular shape.

In particular, the reference color field may for example be used as a reference. Specifically, when determining the analyte concentration within the sample applied to the test field, the color of the reference color field may be used as a reference to be compared to the optically detectable detection reaction of the test chemical with the analyte.

In a further aspect of the invention, a method for measuring an analyte concentration in a sample of blood applied to a test field of an optical test strip by using a mobile device is disclosed. The method comprises the following method steps, which may be performed in the given order. However, a different order may also be possible. Further, one, more than one or even all of the method steps may be performed once or repeatedly. Further, the method steps may be performed successively or, alternatively, two or more method steps may be performed in a timely overlapping fashion or even in parallel. The method may further comprise additional method steps which are not listed.

The method comprises the following steps:

i) providing an optical test strip according to the present invention, such as according to any one of the embodiments disclosed above and/or according to any one of the embodiments disclosed in further detail below;
ii) providing the mobile device, wherein the mobile device comprises at least one camera and at least one wavelength filter, wherein the wavelength filter is configured for essentially blocking transmission of light having wavelengths $\lambda$ of 1200 nm $\geq \lambda \geq WL_{high}$, with 800 nm $\leq WL_{high} \leq$ 1000 nm;
iii) applying the sample of blood to the test field;
iv) capturing at least one image of the test field having the sample applied thereto by using the camera of the mobile device; and
v) determining the analyte concentration of the sample of blood applied to the test field by evaluating an optically detectable detection reaction of a test chemical of the test field.

The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smart phone. Additionally or alternatively, the mobile device may also refer to a tablet computer or another type of portable computer having at least one camera.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, a two-dimensional or even three-dimensional optical information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip. The image itself, thus, may comprise pixels, the pixels of the image correlating to pixels of the camera chip.

The camera specifically may be a color camera. Thus, e.g. for each pixel, color information may be provided or generated, such as color values for three colors R, G, B. A larger number of color values is also feasible, such as four colors for each pixel. Color cameras are generally known to the skilled person. Thus, as an example, each pixel of the camera chip may have three or more different color sensors, such as color recording pixels like one pixel for red (R), one pixel for yellow (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as C, M, Y, K. These techniques are generally known to the skilled person.

The wavelength filter of the mobile device may be integrated into the camera chip, for example into at least one CMOS chip. Thus, specifically when taking images using the camera of the mobile device, light having wavelengths $\lambda$ of 1200 nm $\geq \lambda \geq WL_{high}$ may be essentially blocked. Thus, in particular, when recording images using the mobile device, the wavelength filter with the characterizing wavelength $WL_{high}$ may essentially block light having wavelengths $\lambda$ from being recorded.

For further possible definitions of terms and possible embodiments, reference may be made to the description of the optical test strip given above or as further described below.

The mobile device may further comprise at least one illumination source. The illumination source may specifically be configured for emitting light for the purpose of illuminating an object when taking an image thereof using the mobile device. In particular, the method step iv) may further comprise illuminating the optical test strip, specifically the test field, in particular by using the illumination source of the mobile device.

The optical test strip may, for example, comprises at least one reference color field. In particular, the method may further comprise step vi) of capturing at least one image of the reference color field by using the mobile device, e.g. the camera of the mobile device. Further, the method step vi) may comprise illuminating the optical test strip, specifically the reference color field.

The wavelength filter of the mobile device, in particular, may comprise at least one shortpass filter. As an example, the shortpass filter may specifically have a transmission edge falling with the wavelength of the light. Thus, the shortpass filter may specifically show a rising transmission of light, the lower the wavelength. In particular, the transmission of light through the shortpass filter may fall with rising wavelength. Further, the shortpass filter may have a characteristic wavelength $\lambda_{SP}$. Thus, $WL_{high}$ may equal $\lambda_{SP}$. In particular, as an example, the transmission $T_{SP}$ of the shortpass filter at $\lambda_{SP}$ may be 50 % of a maximum transmission $T_{SPmax}$ of the shortpass filter.

In particular, the characterizing wavelength $WL_{high}$ characterizing the at least one wavelength filter of the mobile device, may for example be in the range of 800 nm $\leq WL_{high} \leq$ 1000 nm, specifically in the range of 800 nm $\leq WL_{high} \leq$ 950 nm, more specifically 800 nm $\leq WL_{high} \leq$ 900 nm.

Specifically, the optical test strip may as an example be or may comprise the optical test strip as disclosed above or as further described below.

In a further aspect of the present invention, a kit for measuring an analyte concentration in a sample of blood is disclosed. The kit comprises an optical test strip according to any one of the embodiments as described above or as described in further detail below and a mobile device comprising at least one camera. The mobile device further comprises at least one wavelength filter, wherein the wavelength filter is configured for essentially blocking transmission of light having wavelengths $\lambda$ of 1200 nm $\geq \lambda \geq WL_{high}$, with 800 nm $\leq WL_{high} \leq$ 1000 nm.

In particular, the mobile device may further comprise at least one illumination source. Specifically, the at least one illumination source of the mobile device may be configured for illuminating an object, such as the optical test strip, when taking an image of the object, e.g. the optical test strip, using the mobile device.

Further, the mobile device may comprise at least one processor. The processor, as an example, may be configured for performing method steps ii) to v) of the method for measuring an analyte concentration in a sample of blood applied to a test field of an optical test strip by using a mobile device, as described above or as further described below. In addition the processor may also be configured for performing method step vi) of the method.

The devices and methods according to the present invention may provide a large number of advantages over known methods and devices for measuring an analyte concentration in a sample of blood. Thus, specifically the present invention may be more independent from ambient lighting conditions than common devices and methods. In particular, as an example the optically detectable detection reaction of the test chemical, specifically the color reaction itself, may vary with spectral range, e.g. may change for different spectral ranges. Thus the optically detectable detection reaction may be dependent on ambient lighting conditions. However, the present invention may provide devices and methods for lessening the impact of ambient light on the optically detectable detection reaction without for example, making use of an additional color filter as hardware element, such as additional filters for a camera. In particular, as an example, when analyzing or evaluating calorimetric color strips, such as for example optical test strips, e.g. for detecting blood glucose, by using a mobile device, e.g. a smartphone camera, in particular, different ambient lighting conditions may need to be compensated and additionally various camera specific properties or characteristics may need to be taken into account. Even, as an example, an illumination source of the mobile device itself may vary for different types and models of mobile devices, e.g. smartphones. Further, as an example, color filters used in RGB channels, such as in camera chips, e.g. in CCD chips or CMOS chips, may differ greatly for various cameras. The present invention may allow an evaluation of the analyte concentration by tracking the intensity change of the test chemical in only a narrowed frequency range. For example, as a result, the evaluation of the analyte concentration may be less dependent on ambient lighting condition, e.g. the lighting situation, and may therefore be more clearly attributed to the observed color reaction, than in known methods and devices.

Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:

Figure 1            shows an embodiment of an optical test for measuring an analyte concentration in a sample of blood in a perspective view;

Figure 2            shows an embodiment of a kit for measuring an analyte concentration in a sample of blood in a perspective view;

Figure 3            shows an embodiment of an optical test strip in a cross sectional view;

Figures 4A and 4B   show different embodiments of a sectional view of an optical test strip;

Figures 5A to 5C    show a graph of transmission spectra of different embodiments of wavelength filter components (Figure 5A) as well as graphs of reflectance spectra of a test chemical (Figure 5B) and of a test field (Figure 5C) for different blood glucose concentrations;

Figures 6A and 6B   show graphs of histograms of the red color recorded by an embodiment of a mobile device having a camera; and

Figures 7A and 7B   show flow charts of different embodiments of a method for measuring an analyte concentration in a sample of blood applied to a test field of an optical test strip by using a mobile device.

Detailed description of the embodiments

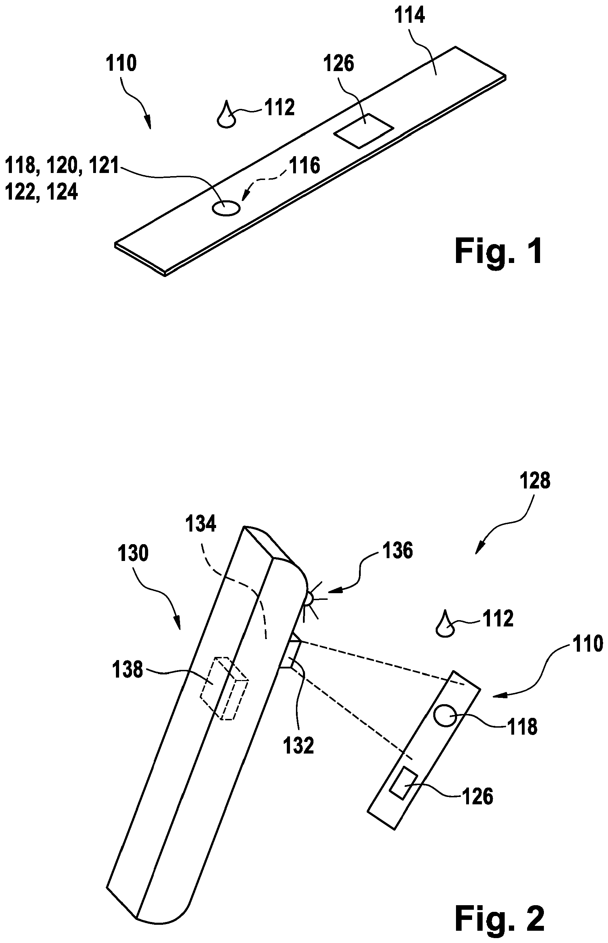

In Figure 1 an embodiment of an optical test strip 110 for measuring an analyte concentration in a sample 112 of blood is illustrated in a perspective view. The optical test strip 110 comprises a test strip carrier 114 having at least one transparent area 116. The optical test strip 110 further comprises at least one test field 118 comprising at least one carrier foil 120 applied to the test strip carrier 114, the carrier foil 120 having at least one wavelength filter component 121. The test field 118 further comprising at least one test chemical 122 applied to the carrier foil 120, the test chemical 122 being configured for performing an optically detectable detection reaction with the analyte, and at least one porous material 124 for at least partially filtering out solid components contained in the sample 112. Further, the optical test strip 110 may comprise at least one reference color field 126.

Figure 2 shows an embodiment of a kit 128 for measuring an analyte concentration in a sample 112 of blood in a perspective view. The kit 128 comprises an optical test strip 110 and a mobile device 130. The mobile device 130 comprises at least one camera 132 and at least one wavelength filter 134. Further, the mobile device 130 may comprise at least one illumination source 136 and at least one processor 138.

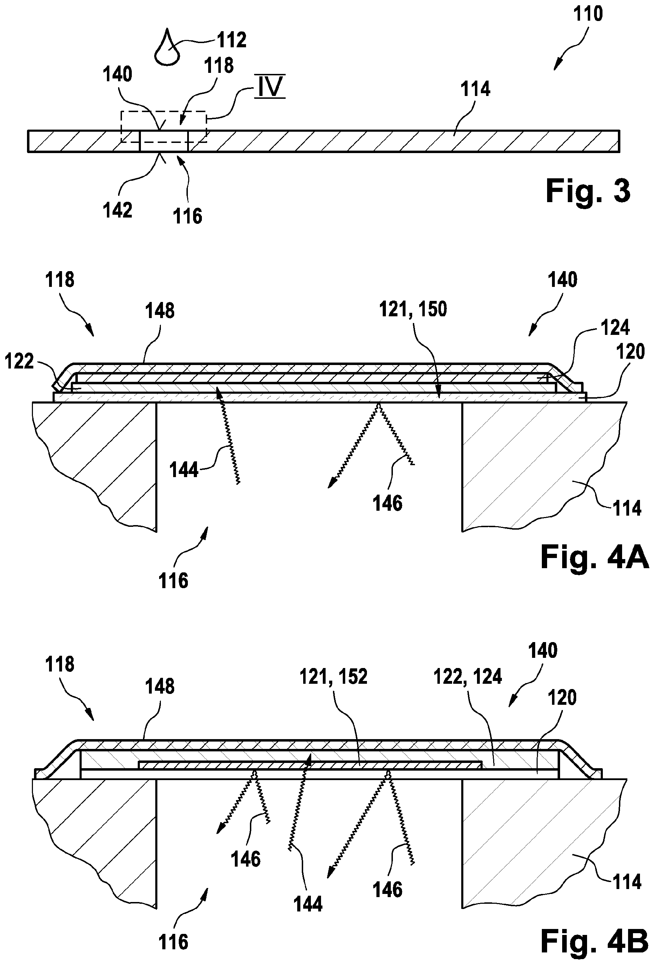

In Figure 3 an embodiment of an optical test strip 110 is illustrated in a cross sectional view. In particular, as illustrated in Figure 3, the sample 112 may be applied to a first side 140 of the optical test strip 110, specifically of the test field 118, wherein the optically detectable detection reaction of the test chemical 122 may be evaluated from a second side 142 of the optical test strip 110, specifically of the test field 118. In Figures 4A and 4B different embodiments of a sectional view of an optical test strip 110 are illustrated. Figures 4A and 4B may specifically show embodiments of enlarged sectional views of a part of the optical test strip 110 indicated by area IV illustrated in Figure 3. Specifically, Figures 4A and 4B may illustrate different embodiments of the test field 118.

As illustrated in Figures 4A and 4B, the carrier foil 120 of the test field 118 is applied to the test strip carrier 114 and at least partially covers the transparent area 116 of the test strip carrier 114. The test field 118 further comprises the test chemical 122 configured for performing the optically detectable detection reaction. The test chemical 122 is further configured for at least partially absorbing light having at least one absorption wavelength $\lambda_{abs}$ 144, wherein the at least one absorption wavelength $\lambda_{abs}$ 144 is in the range 650 nm < $\lambda_{abs}$ ≤ 1100 nm. Further, as illustrated in Figure 4A, the test field 118 comprises the at least one porous material 124 for at least partially filtering out solid components contained in the sample 112. The test chemical 122 and the at least partially porous material 124 may be arranged in separate layers, as for example illustrated in Figure 4A. In particular, as illustrated in Figure 4B, the test chemical 122 and the at least partially porous material 124 may also be combined into one layer. Further, the test field 118 may comprise a spreading layer 148, configured to equally spread or distribute the sample 112 of blood over a surface, specifically over the first side 140, of the test field 118 on which the sample 112 is applied.

The carrier foil 120 further has the at least one wavelength filter component 121 which is adapted to essentially block light having $\lambda_{blc}$ of 10 nm ≤ $\lambda_{blc}$ ≤ $WL_{low}$, with 550 nm ≤ $WL_{low}$ ≤ 650 nm. In particular, the wavelength filter component 121 may for example be located within the carrier foil 120, specifically the wavelength filter component 121 may be dispersed within the carrier foil 120, as for example illustrated in Figure 4A. More particularly, the wavelength filter component 121 may be or may comprise at least one longpass filter 150. Additionally or alternatively, the wavelength filter component 121 may be or may comprise at least one interference filter 152. Specifically, the interference filter 152 may be located on at least one surface of the carrier foil 120, specifically, as illustrated in Figure 4B, on an upper surface of the carrier foil 120. However, the interference filter 152 may also be applied on a lower surface of the carrier foil 120 or on both an upper and a lower surface of the carrier foil 120.

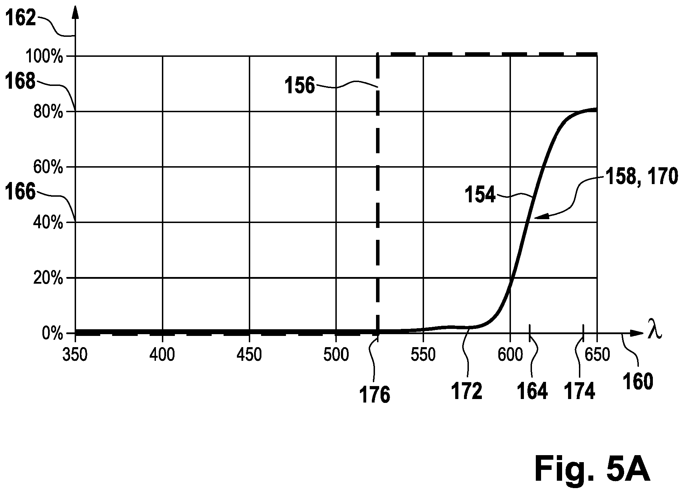

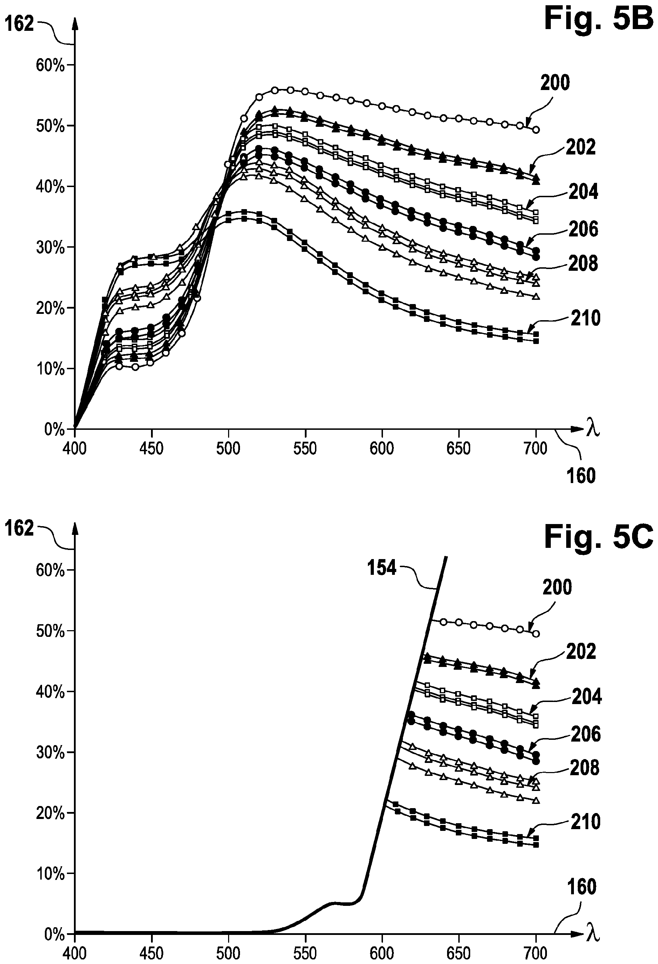

Figure 5A illustrates a graph of transmission spectra of different embodiments of wavelength filter components 121. In particular, as an example, a first transmission spectrum 154 of a longpass filter 150 and a second transmission spectrum 156 of an interference filter 152 may be illustrated. Specifically, as illustrated in Figure 5A, the longpass filter may have a transmission edge 158 rising with the wavelength of the light shown on the x-axis 160. The y-axis 162 may specifically show the transmission of the wavelength filter components 121. Further, the longpass filter 150 may have a characteristic wavelength $\lambda_{LP}$ 164, wherein a transmission $T_{LP}$ 166 of the longpass filter 150 at $\lambda_{LP}$ 164 may be 50 % of a maximum transmission $T_{LPmax}$ 168 of the longpass filter 150. Thus, in Figure 5, as an example, $T_{LPmax}$ 168 may be 80 % and therefore $T_{LP}$ 166 may be 0.5 x 80 % = 40 %. Further, the longpass filter 150 may have a steepness $S_{LP}$ 170 of the rising transmission edge 158. In particular, as described in Equation (1) above, $S_{LP}$ may be calculated by using wavelengths $\lambda_{blc}$ 172 and $\lambda_{trans}$ 174. Specifically, at wavelength $\lambda_{blc}$ 172 the Transmission $T_{LP}$ of the longpass filter 150 may be equal to or smaller than 5 % of $T_{LPmax}$ and at wavelength $\lambda_{trans}$ 174 the Transmission $T_{LP}$ of the longpass filter 150 may be equal to or greater than 95 % of $T_{LPmax}$.

Further, the interference filter 152 may have a characteristic wavelength $\lambda_{HPF}$ 176, wherein, as illustrated by the second transmission spectrum 156 in Figure 5, the interference filter 152 may selectively block or attenuate all light having wavelengths below $\lambda_{HPF}$ 176 and wherein the interference filter 152 may transmit all light having wavelengths higher than $\lambda_{HPF}$ 176.

As an example, the wavelength filter component 121 may specifically allow an intensity based evaluation of the optical test strip 110, specifically of the optically detectable detection reaction of the test chemical 122, as an example, independently from ambient light and characteristics of the mobile device 130, specifically characteristics of the camera 132.

Figure 5B shows a graph of reflection spectra of the test chemical 122 for different blood glucose concentrations, wherein no wavelength filter component 121 is present in the carrier foil 120 of the test field 118. In particular, the graph may illustrate the reflection spectra of various test fields comprising the test chemical 122 but having no wavelength filter component 121. For example, the illustrated values of reflection may occur 20s after contact with the test chemical 122. Specifically, the different blood glucose concentrations may vary with the wavelength of the light shown on the x-axis 160, wherein the y-axis 132 may show the relative reflectance, specifically a percentage of the reflectance, for the different blood glucose concentrations. In particular, six different blood glucose concentrations may be illustrated by six different curves in the graph. Specifically, curves 200 may illustrate the reflection spectrum of a blood glucose concentration of 0 milligrams per deciliter (mg/dl), curves 202 may illustrate the reflection spectrum of a blood glucose concentration of 40 mg/dl, curves 204 may illustrate the reflection spectrum of a blood glucose concentration of 80 mg/dl, curves 206 may illustrate the reflection spectrum of a blood glucose concentration of 160 mg/dl, curves 208 may illustrate the reflection spectrum of a blood glucose concentration of 260 mg/dl and curves 210 may illustrate the reflection spectrum of a blood glucose concentration of 440 mg/dl.

Figure 5C, shows a graph of a reflection spectra of an embodiment of the test field 118 for the different blood glucose concentrations. Specifically, the test field 118 comprises the carrier foil 120 having the wavelength filter component 121. Thus, the reflection spectra shown in Figure 5C may illustrate the reflection spectra of the test chemical 122 for different blood glucose concentrations, wherein light having wavelengths $\lambda_{blc}$ (172) may be essentially blocked by the wavelength filter component 121 of the carrier foil 120 of the test field 118. As an example, the reflection spectra shown in Figure 5C may illustrate the reflection spectra of the same blood glucose concentrations used for Figure 5B, wherein the reflection spectra is superposed by the first transmission spectrum 154 of the longpass filter 150, as illustrated in Figure 5A. For better understanding, a part of the transmission spectrum 154 of the longpass filter 150 is additionally illustrated in Figure 5C.

As illustrated, the different blood glucose concentrations may be separated clearly and may show linear courses for wavelengths $\lambda \geq 550$nm, specifically for $\lambda \geq 600$nm. Thus, determining an analyte concentration such as the blood glucose concentration, of a sample 112 applied to the test field 118 of an optical test strip 110 according to the present invention, may particularly improve a measurement accuracy of the analyte concentration. Specifically, since, as illustrated, a determination of an analyte concentration may be difficult in a wavelength-range, for example for wavelengths $\lambda < 550$nm, in which the analyte concentration, e.g. blood glucose concentration, significantly varies for very small changes of lighting conditions.

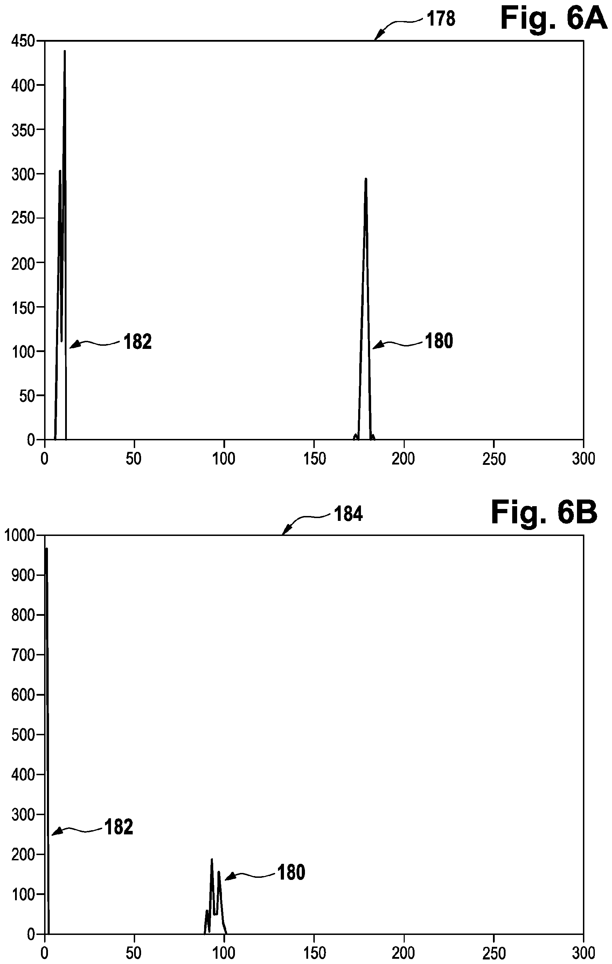

In Figures 6A and 6B show graphs of histograms of the red color recorded by an embodiment of a mobile device 130 having a camera 132. As an example, in Figure 6A, a signal, specifically a signal of the red channel of the camera 132, may be illustrated in a first histogram 178, specifically when taking an image without using a wavelength filter 134. In particular, for example in the first histogram 178 a first illumination 180, specifically white standard light, for example having 6500 K and a second illumination 182, specifically greenish light, may have been used for ambient lighting. As an example, it may be seen that with the second illumination 182, e.g. with the greenish light, as an example, a faulty signal of about 10 counts may be recorded. However, in Figure 6B, a signal of the red channel of the camera 132, may be illustrated in a second histogram 184, for example analogous to the first histogram shown in Figure 6A, wherein in the second histogram 184 a red filter may be used. It may, for example, be seen that the second illumination 182, e.g. the "greenish" lighting, specifically may no longer result in a faulty signal. As an example, the signal of the first illumination 180, e.g. of the white light, when comparing the first histogram 178 to the second histogram 184, may for example differ by about 100 Counts. This may for example appear due to losses within the filter. However, as an example, the signal may be amplified and/or raised, for example, to 180 counts, such as by using stronger illumination.

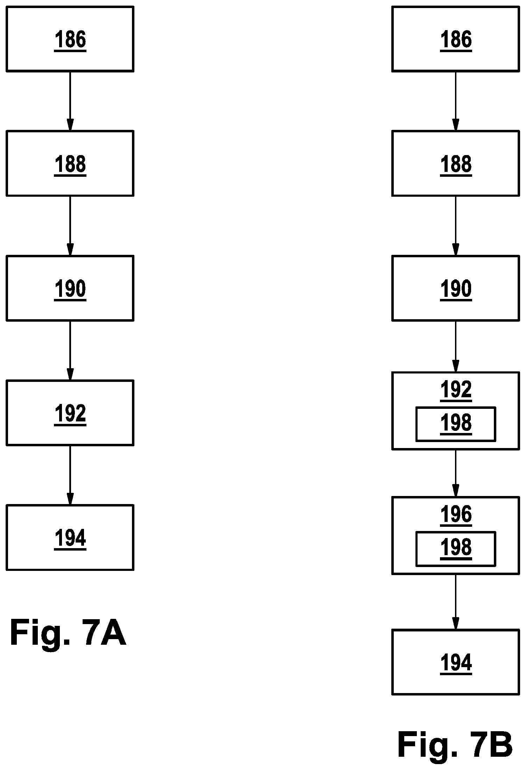

Figure 7A shows a flow chart of an embodiment of a method for measuring an analyte concentration in a sample 112 of blood applied to a test field 118 of an optical test strip 110 by using a mobile device 130. The method comprises the following steps:

i) (denoted with reference number 186) providing an optical test strip 110 having at least one wavelength filter component 121 which is adapted to essentially block light having wavelengths $\lambda_{blc}$ 146 of 10 nm $\leq \lambda_{blc} \leq WL_{low}$, with 550 nm $\leq WL_{low} \leq$ 650 nm;

ii) (denoted with reference number 188) providing the mobile device 130, wherein the mobile device 130 comprises at least one camera 132 and at least one wavelength filter 134, wherein the wavelength filter 134 is configured for essentially blocking transmission of light having wavelengths $\lambda$ of 1200 nm $\geq \lambda \geq WL_{high}$, with 800 nm $\leq WL_{high} \leq$ 1000 nm;

iii) (denoted with reference number 190) applying the sample 112 of blood to the test field 118;

iv) (denoted with reference number 192) capturing at least one image of the test field 118 having the sample 112 applied thereto by using the camera 132 of the mobile device 130; and

v) (denoted with reference number 194) determining the analyte concentration of the sample 112 of blood applied to the test field 118 by evaluating an optically detectable detection reaction of a test chemical 122 of the test field 118.

Figure 7B shows a flow chart of another embodiment of a method for measuring an analyte concentration in a sample 112 of blood applied to a test field 118 of an optical test strip 110 by using a mobile device 130. As illustrated in Figure 7B, the method may further comprise step vi) (denoted with reference number 196) capturing at least one image of the reference color field 126 by using the mobile device 130. As an example, step vi) may specifically be performed before performing step v). In particular, step iv) and/or step vi) may further comprise a substep 198 of illuminating the optical test strip 110, specifically by using the illumination source 136 of the mobile device 130.

List of reference numbers

[0078]

| | |
|---|---|
| 110 | optical test strip |
| 112 | Sample |
| 114 | test strip carrier |
| 116 | transparent area |
| 118 | test field |
| 120 | carrier foil |
| 121 | wavelength filter component |
| 122 | test chemical |
| 124 | porous material |
| 126 | reference color field |
| 128 | Kit |
| 130 | mobile device |
| 132 | Camera |
| 134 | wavelength filter |
| 136 | illumination source |
| 138 | processor |
| 140 | first side |
| 142 | second side |
| 144 | $\lambda_{abs}$ |
| 146 | $\lambda_{blc}$ |
| 148 | spreading layer |
| 150 | longpass filter |
| 152 | interference filter |
| 154 | first transmission spectrum |
| 156 | second transmission spectrum |
| 158 | transmission edge |
| 160 | x-axis |
| 162 | y-axis |
| 164 | $\lambda_{LP}$ |
| 166 | $T_{LP}$ |
| 168 | $T_{LPmax}$ |
| 170 | $S_{LP}$ |
| 172 | $\lambda_{blc}$ |
| 174 | $\lambda_{trans}$ |
| 176 | $\lambda_{HPF}$ |
| 178 | first histogram |
| 180 | first illumination |
| 182 | second illumination |
| 184 | second histogram |
| 186 | step i) |
| 188 | step ii) |
| 190 | step iii) |
| 192 | step iv) |
| 194 | step v) |
| 196 | step vi) |
| 198 | illuminating the test strip |
| 200 | 0 mg/dl curve |
| 202 | 40 mg/dl curve |
| 204 | 80 mg/dl curve |
| 206 | 160 mg/dl curve |
| 208 | 260 mg/dl curve |
| 210 | 440 mg/dl curve |

**Claims**

1. An optical test strip (110) for measuring an analyte concentration in a sample (112) of blood, comprising:

   a) a test strip carrier (114) having at least one transparent area (116);
   b) a test field (118), wherein the test field (118):

   - comprises at least one carrier foil (120), wherein the carrier foil (120) is applied to the test strip carrier (114) and at least partially covers the transparent area (116) of the test strip carrier (114);
   - comprises at least one test chemical (122) applied to the carrier foil (120), the test chemical (122) being configured for performing an optically detectable detection reaction with the analyte, wherein the test chemical (122) is further configured for at least partially absorbing light having at least one absorption wavelength $\lambda_{abs}$ (144) in the range 650 nm < $\lambda_{abs}$ ≤ 1100 nm; and
   - comprises at least one porous material (124) for at least partially filtering out solid components contained in the sample (112);
   wherein the carrier foil (120) has at least one wavelength filter component (121) which is adapted to essentially block light having wavelengths $\lambda_{blc}$ (146) of 10 nm ≤ $\lambda_{blc}$ ≤ $WL_{low}$, with 550 nm ≤ $WL_{low}$ ≤ 650 nm.

2. The optical test strip (110) according to any one of the preceding claims, wherein the wavelength filter component (121) is located within the carrier foil (120).

3. The optical test strip (110) according to any one of the preceding claims, wherein the wavelength filter component (121) is selected from the group consisting of a longpass filter component and a bandpass filter component.

4. The optical test strip (110) according to any one of the preceding claims, wherein the wavelength filter component (121) comprises at least one longpass filter (150), wherein the longpass filter (150) has a transmission edge (158) rising with the wavelength of the light, wherein the longpass filter (150) further has a characteristic wavelength $\lambda_{LP}$ (164), wherein a transmission of the longpass filter (150) at $\lambda_{LP}$ (164) is 50 % of a maximum transmission of the longpass filter (150) and wherein $WL_{low} = \lambda_{LP}$ (164).

5. The optical test strip (110) according to any one of the preceding claims, wherein the wavelength filter component (121) comprises an interference filter (152), wherein the interference filter (152) has a characteristic wavelength $\lambda_{HPF}$ (176), wherein $WL_{low} = \lambda_{HPF}$ (176), wherein the interference filter (152) preferably is located on at least one surface of the carrier foil (120).

6. The optical test strip (110) according to any one of the preceding claims, wherein the optical test strip (110), specifically the carrier foil (120), comprises at least one further filter component, wherein the at least one further filter component comprises a shortpass filter, wherein the further filter component preferably is configured for essentially blocking transmission of light having wavelengths $\lambda \geq WL_{high}$, with $WL_{high} > WL_{low}$.

7. A method for measuring an analyte concentration in a sample (112) of blood applied to a test field (118) of an optical test strip (110) by using a mobile device (130), comprising:

   i) providing an optical test strip (110) according to any one of the preceding claims;
   ii) providing the mobile device (130), wherein the mobile device (130) comprises at least one camera (132) and at least one wavelength filter (134), wherein the wavelength filter (134) is configured for essentially blocking transmission of light having wavelengths $\lambda$ of 1200 nm ≥ $\lambda$ ≥ $WL_{high}$, with 800 nm ≤ $WL_{high}$ ≤ 1000 nm;
   iii) applying the sample (112) of blood to the test field (118);
   iv) capturing at least one image of the test field (118) having the sample (112) applied thereto by using the camera (132) of the mobile device (130); and
   v) determining the analyte concentration of the sample (112) of blood applied to the test field (118) by evaluating an optically detectable detection reaction of a test chemical (122) of the test field (118).

8. The method according to any one of the preceding method claims, wherein the mobile device (130) further comprises at least one illumination source (136), wherein method step iv) further comprises illuminating the optical test strip (110).

9. The method according to any one of the preceding method claims, wherein the wavelength filter (134) comprises at least one shortpass filter wherein the shortpass filter has a transmission edge falling with the wavelength of the light, wherein the shortpass filter further has a characteristic wavelength $\lambda_{SP}$, wherein a transmission of the shortpass filter at $\lambda_{SP}$ is 50 % of a maximum transmission of the shortpass filter and wherein $WL_{high} = \lambda_{SP}$.

10. The method according to any one of the preceding method claims, wherein 800 nm $\leq WL_{high} \leq$ 950 nm, specifically 800 nm $\leq WL_{high} \leq$ 900 nm.

11. The method according to any one of the preceding method claims, wherein the optical test strip (110) comprises an optical test strip (110) according to any one of the preceding claims referring to an optical test strip (110).

12. A kit (128) for measuring an analyte concentration in a sample (112) of blood, the kit (128) comprising an optical test strip (110) according to any one of the preceding claims referring to an optical test strip (110) and the kit further comprising a mobile device (130), wherein the mobile device (130) comprises at least one camera (132), wherein the mobile device (130) further comprises at least one wavelength filter (134), wherein the wavelength filter (134) is configured for essentially blocking transmission of light having wavelengths $\lambda$ of 1200 nm $\geq \lambda \geq WL_{high}$, with 800 nm $\leq WL_{high} \leq$ 1000 nm.

**Patentansprüche**

1. Optischer Teststreifen (110) zum Messen einer Analytkonzentration in einer Blutprobe (112), umfassend:

   a) einen Teststreifenträger (114) mit wenigstens einem transparenten Bereich (116);
   b) ein Testfeld (118), wobei das Testfeld (118):

   - wenigstens eine Trägerfolie (120) umfasst, wobei die Trägerfolie (120) auf den Teststreifenträger (114) aufgebracht ist und den transparenten Bereich (116) des Teststreifenträgers (114) wenigstens teilweise bedeckt;
   - wenigstens eine Testchemikalie (122) umfasst, die auf die Trägerfolie (120) aufgebracht ist, wobei die Testchemikalie (122) eingerichtet ist, eine optisch nachweisbare Nachweisreaktion mit dem Analyten durchzuführen, wobei die Testchemikalie (122) ferner eingerichtet ist, Licht mit wenigstens einer Absorptionswellenlänge $\lambda_{abs}$ (144) in dem Bereich von 650 nm < $\lambda_{abs} \leq$ 1100 nm wenigstens teilweise zu absorbieren; und
   - wenigstens ein poröses Material (124) zum wenigstens teilweisen Herausfiltrieren von in der Probe (112) enthaltenen festen Komponenten umfasst;
   wobei die Trägerfolie (120) wenigstens eine Wellenlängenfilterkomponente (121) aufweist, die dafür ausgelegt ist, Licht mit Wellenlängen von $\lambda_{blc}$ (146) von 10 nm $\leq \lambda_{blc} \leq WL_{tief}$, wobei 550 nm $\leq WL_{tief} \leq$ 650 nm, weitgehend zu blockieren.

2. Optischer Teststreifen (110) gemäß einem der vorstehenden Ansprüche, wobei die Wellenlängenfilterkomponente (121) innerhalb der Trägerfolie (120) angeordnet ist.

3. Optischer Teststreifen (110) gemäß einem der vorstehenden Ansprüche, wobei die Wellenlängenfilterkomponente (121) ausgewählt ist aus der Gruppe bestehend aus einer Langpassfilterkomponente und einer Bandpassfilterkomponente.

4. Optischer Teststreifen (110) gemäß einem der vorstehenden Ansprüche, wobei die Wellenlängenfilterkomponente (121) wenigstens einen Langpassfilter (150) umfasst, wobei der Langpassfilter (150) eine Transmissionskante (158) aufweist, die mit der Wellenlänge des Lichts ansteigt, wobei der Langpassfilter (150) ferner eine charakteristische Wellenlänge $\lambda_{LP}$ (164) aufweist, wobei eine Transmission des Langpassfilters (150) bei $\lambda_{LP}$ (164) 50 % einer höchsten Transmission des Langpassfilters (150) beträgt und wobei $WL_{tief} = \lambda_{LP}$ (164).

5. Optischer Teststreifen (110) gemäß einem der vorstehenden Ansprüche, wobei die Wellenlängenfilterkomponente (121) einen Interferenzfilter (152) umfasst, wobei der Interferenzfilter (152) eine charakteristische Wellenlänge $\lambda_{HPF}$ (176) aufweist, wobei $WL_{tief} = \lambda_{HPF}$ (176), wobei der Interferenzfilter (152) vorzugsweise an wenigstens einer Oberfläche der Trägerfolie (120) angeordnet ist.

6. Optischer Teststreifen (110) gemäß einem der vorstehenden Ansprüche, wobei der optische Teststreifen (110), insbesondere die Trägerfolie (120), wenigstens eine weitere Filterkomponente umfasst, wobei die wenigstens eine weitere Filterkomponente einen Kurzpassfilter umfasst, wobei die weitere Filterkomponente vorzugsweise eingerichtet ist, Transmission von Licht mit Wellenlängen von $\lambda \geq WL_{hoch}$, wobei $WL_{hoch} > WL_{tief}$, weitgehend zu blockieren.

7. Verfahren zum Messen einer Analytkonzentration in einer Blutprobe (112), die auf ein Testfeld (118) eines optischen Teststreifens (110) aufgebracht ist, unter Verwendung einer mobilen Vorrichtung (130), umfassend:

   i) Bereitstellen eines optischen Teststreifens (110) gemäß einem der vorstehenden Ansprüche;
   ii) Bereitstellen der mobilen Vorrichtung (130), wobei die mobile Vorrichtung (130) wenigstens eine Kamera (132) und wenigstens einen Wellenlängenfilter (134) umfasst, wobei der Wellenlängenfilter (134) eingerichtet ist, Transmission von Licht mit Wellenlängen $\lambda$ von 1200 nm $\geq \lambda \geq WL_{hoch}$, wobei 800 nm $\leq WL_{hoch} \leq$ 1000 nm, weitgehend zu blockieren;
   iii) Aufbringen der Blutprobe (112) auf das Testfeld (118);
   iv) Aufnehmen wenigstens eines Bilds des Testfelds (118) mit der darauf aufgebrachten Probe (112) unter Verwendung der Kamera (132) der mobilen Vorrichtung (130); und
   v) Bestimmen der Analytkonzentration der auf das Testfeld (118) aufgebrachten Blutprobe (112) durch Auswerten einer optisch nachweisbaren Nachweisreaktion einer Testchemikalie (122) des Testfelds (118).

8. Verfahren gemäß einem der vorstehenden Verfahrensansprüche, wobei die mobile Vorrichtung (130) ferner wenigstens eine Beleuchtungsquelle (136) umfasst, wobei der Verfahrensschritt iv) ferner Beleuchten des optischen Teststreifens (110) umfasst.

9. Verfahren gemäß einem der vorstehenden Verfahrensansprüche, wobei der Wellenlängenfilter (134) wenigstens einen Kurzpassfilter umfasst, wobei der Kurzpassfilter eine Transmissionskante aufweist, die mit der Wellenlänge des Lichts fällt, wobei der Kurzpassfilter ferner eine charakteristische Wellenlänge $\lambda_{SP}$ aufweist, wobei eine Transmission des Kurzpassfilters bei $\lambda_{SP}$ 50 % einer höchsten Transmission des Kurzpassfilters beträgt und wobei $WL_{hoch} = \lambda_{SP}$.

10. Verfahren gemäß einem der vorstehenden Verfahrensansprüche, wobei 800 nm $\leq WL_{hoch} \leq$ 950 nm, insbesondere 800 nm $\leq WL_{hoch} \leq$ 900 nm.

11. Verfahren gemäß einem der vorstehenden Verfahrensansprüche, wobei der optische Teststreifen (110) einen optischen Teststreifen (110) gemäß einem der vorstehenden Ansprüche, die einen optischen Teststreifen (110) betreffen, umfasst.

12. Kit (128) zum Messen einer Analytkonzentration in einer Blutprobe (112), wobei das Kit (128) einen optischen Teststreifen (110) gemäß einem der vorstehenden Ansprüche, die einen optischen Teststreifen (110) betreffen, umfasst und das Kit ferner eine mobile Vorrichtung (130) umfasst, wobei die mobile Vorrichtung (130) wenigstens eine Kamera (132) umfasst, wobei die mobile Vorrichtung (130) ferner wenigstens einen Wellenlängenfilter (134) umfasst, wobei der Wellenlängenfilter (134) eingerichtet ist, Transmission von Licht mit Wellenlängen $\lambda$ von 1200 nm $\geq \lambda \geq WL_{hoch}$, wobei 800 nm $\leq WL_{hoch} \leq$ 1000 nm, weitgehend zu blockieren.

## Revendications

1. Bandelette d'essai optique (110) pour mesurer une concentration d'analyte dans un échantillon (112) de sang, comprenant :

   a) un support de bandelette d'essai (114) ayant au moins une zone transparente (116) ;
   b) un champ d'essai (118), le champ d'essai (118) :

      - comprenant au moins une feuille de support (120), la feuille de support (120) étant appliquée sur le support de bandelettes d'essai (114) et recouvrant au moins partiellement la zone transparente (116) du support de bandelettes d'essai (114) ;
      - comprenant au moins un produit chimique d'essai (122) appliqué sur la feuille de support (120), le produit chimique d'essai (122) étant configuré pour réaliser une réaction de détection optiquement détectable avec l'analyte, le produit chimique d'essai (122) étant en outre configuré pour absorber au moins partiellement la lumière présentant au moins une longueur d'onde d'absorption $\lambda_{abs}$ (144) dans la plage 650 nm < $\lambda_{abs}$ $\leq$ 1100 nm ; et

- comprenant au moins un matériau poreux (124) pour filtrer au moins partiellement les composants solides contenus dans l'échantillon (112) ;

la feuille de support (120) présentant au moins un composant de filtre de longueur d'onde (121) qui est adapté pour bloquer essentiellement la lumière ayant des longueurs d'onde $\lambda_{blc}$ (146) de 10 nm $\leq \lambda_{blc} \leq$ $WL_{low}$, avec 550 nm $\leq WL_{low} \leq$ 650 nm.

2. Bandelette d'essai optique (110) selon l'une quelconque des revendications précédentes, le composant de filtre de longueur d'onde (121) étant situé à l'intérieur de la feuille de support (120).

3. Bandelette d'essai optique (110) selon l'une quelconque des revendications précédentes, le composant de filtre de longueur d'onde (121) étant choisi dans le groupe constitué par un composant de filtre passe-bas et un composant de filtre passe-bande.

4. Bandelette d'essai optique (110) selon l'une quelconque des revendications précédentes, le composant de filtre de longueur d'onde (121) comprenant au moins un filtre passe-bas (150), le filtre passe-bas (150) ayant un bord de transmission (158) qui monte avec la longueur d'onde de la lumière, le filtre passe-bas (150) ayant en outre une longueur d'onde caractéristique $\lambda_{Lp}$ (164), une transmission du filtre passe-bas (150) à $\lambda_{LP}$ (164) étant de 50 % d'une transmission maximale du filtre passe-bas (150) et $WL_{low} = \lambda_{LP}$ (164) .

5. Bandelette d'essai optique (110) selon l'une quelconque des revendications précédentes, le composant de filtre de longueur d'onde (121) comprenant un filtre d'interférence (152), le filtre d'interférence (152) ayant une longueur d'onde caractéristique $\lambda_{HPF}$ (176), $WL_{low} = \lambda_{HPF}$, le filtre d'interférence (152) étant de préférence situé sur au moins une surface de la feuille de support (120).

6. Bandelette d'essai optique (110) selon l'une quelconque des revendications précédentes, la bandelette d'essai optique (110), en particulier la feuille de support (120), comprenant au moins un composant de filtre supplémentaire, l'au moins un composant de filtre supplémentaire comprenant un filtre passe-haut, le composant de filtre supplémentaire étant de préférence configuré pour bloquer essentiellement la transmission de la lumière ayant des longueurs d'onde $\lambda \geq WL_{high}$, avec $WL_{high} > WL_{low}$.

7. Procédé pour mesurer une concentration d'analyte dans un échantillon (112) de sang appliqué sur un champ d'essai (118) d'une bandelette d'essai optique (110) en utilisant un dispositif mobile (130), comprenant :

   i) la fourniture d'une bandelette d'essai optique (110) selon l'une quelconque des revendications précédentes ;
   ii) la fourniture du dispositif mobile (130), le dispositif mobile (130) comprenant au moins une caméra (132) et au moins un filtre de longueur d'onde (134), le filtre de longueur d'onde (134) étant configuré pour essentiellement bloquer la transmission de la lumière ayant des longueurs d'onde $\lambda$ de 1200 nm $\geq \lambda \geq WL_{high}$, avec 800 nm $\leq$ $WL_{high} \leq$ 1000 nm ;
   iii) l'application de l'échantillon (112) de sang sur le champ d'essai (118) ;
   iv) la capture d'au moins une image du champ d'essai (118) sur lequel est appliqué l'échantillon (112) en utilisant la caméra (132) du dispositif mobile (130) ; et
   v) la détermination de la concentration de l'analyte de l'échantillon (112) de sang appliqué sur le champ d'essai (118) en évaluant une réaction de détection optiquement détectable d'un produit chimique d'essai (122) du champ d'essai (118).

8. Procédé selon l'une quelconque des revendications précédentes se référant à un procédé, le dispositif mobile (130) comprenant en outre au moins une source d'éclairage (136), l'étape iv) du procédé comprenant en outre l'éclairage de la bandelette d'essai optique (110) .

9. Procédé selon l'une quelconque des revendications précédentes se référant à un procédé, le filtre de longueur d'onde (134) comprenant au moins un filtre passe-haut, le filtre passe-haut ayant un bord de transmission qui diminue avec la longueur d'onde de la lumière, le filtre passe-haut ayant en outre une longueur d'onde caractéristique $\lambda_{SP}$, une transmission du filtre passe-haut à $\lambda_{SP}$ étant de 50 % d'une transmission maximale du filtre passe-haut et $WL_{high} = \lambda_{SP}$.

10. Procédé selon l'une quelconque des revendications précédentes se référant à un procédé, 800 nm $\leq WL_{high} \leq$ 950 nm, plus précisément 800 nm $\leq WL_{high} \leq$ 900 nm.

**11.** Procédé selon l'une quelconque des revendications précédentes se référant à un procédé, la bandelette d'essai optique (110) comprenant une bandelette d'essai optique (110) selon l'une quelconque des revendications précédentes se référant à une bandelette d'essai optique (110).

**12.** Kit (128) pour mesurer une concentration d'analyte dans un échantillon (112) de sang, le kit (128) comprenant une bandelette d'essai optique (110) selon l'une quelconque des revendications précédentes se référant à une bandelette d'essai optique (110) et le kit comprenant en outre un dispositif mobile (130), le dispositif mobile (130) comprenant au moins une caméra (132), le dispositif mobile (130) comprenant en outre au moins un filtre de longueur d'onde (134), le filtre de longueur d'onde (134) étant configuré pour bloquer essentiellement la transmission de la lumière ayant des longueurs d'onde $\lambda$ de 1200 nm $\geq \lambda \geq$ WL$_{high}$, avec 800 nm $\leq$ WL$_{high}$ $\leq$ 1000 nm.

**Fig. 1**

**Fig. 2**

110

112

140   118   IV

114

142   116

**Fig. 3**

118   148   121, 150   140   124   120

122

144   146   114

116

**Fig. 4A**

118   148   121, 152   122, 124   140   120

146   144   146   114

116

**Fig. 4B**

**Fig. 5A**

Fig. 5B

Fig. 5C

Fig. 6A

Fig. 6B

Fig. 7A

Fig. 7B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170343480 A1

- US 2006051738 A1

**Non-patent literature cited in the description**

- **HONES et al.** *Diabetes Technology and Therapeutics,* 2008, vol. 10 (1), 10-26